# EUROPEAN PATENT APPLICATION

(11) **EP 1 679 301 A1**
(43) Date of publication of application: **12.07.2006**
(21) Application number: 05460024.2
(22) Date of filing: 30.08.2005
(51) Int. Cl.: C07C 69/675, C07C 69/738, C07C 69/734, C07C 69/736, C07C 69/88, C07C 229/42, C07D 209/28, C07C 317/44, C07D 207/32, A61K 31/216, A61K 31/235, A61K 31/405, A61K 31/40, A61P 29/00, C08G 63/08

(54) **Esters of nonsteroidal anti-inflammatory drugs and methods of their preparation**

(30) Priority: 11.01.2005 PL 37218105
(71) Applicant: Centrum Chemii Polimerow Pan, 41-819 Zabrze (PL)
(72) Inventor: Jedlinsky, Zbigniew, 44-100 Gliwice (PL); Luczyk-Juzwa, Maria, 41-800 Zabrze (PL); Zawidlak-Wegrzynska, 41-908 Bytom (PL); Kaczmarczyk, Bozena, 41-907 Bytom (PL); Bosek, Izabele, 41-807 Zabrze (PL); Wanic, Andrzej, 41-200 Sosnowiec (PL)
(74) Representative: Brodowska, Iwona

(57) **Abstract**

Novel esters of nonsteroidal anti-inflammatory drugs with oligomers of 3-hydroxybutyric acid of the invention possess the general formula 1, where n from 2 to 35, and R is a radical of a non-steroidal anti-inflammatory drug selected from the group consisting of ibuprofen, ketoprofen, fenoprofen, naproxen, diclofenac, indomethacine, sulindac, tolmetin or salicylic acid.

The method of preparation of compounds of the formula 1 consists in converting nonsteroidal anti-inflammatory drugs into a monosodium or monopotassium salts in the presence of a strongly polar solvent, preferably dimethyl sulfoxide (DMSO) or dimethylformamide (DMF), and in subjecting the obtained salts to the reaction with β-butyrolactone to open the lactone ring and to polymerize the lactone.

The esters of the invention contain a nonsteroidal anti-inflammatory drugs bonded by means of an ester bond with oligomers of 3-hydroxybutyric acid including 2-35 mers as constituting biocompatible polymer carriers.

## Description

The presented invention provides novel esters of nonsteroidal anti-inflammatory drugs (NSAIDs) selected from the following group: ibuprofen, ketoprofen, naproxen, diclofenac, indomethacine, sulindac, tolmetin, fenoprofen, or salicylic acid, as well as the methods of their preparation and medical applications.

Typical nonsteroidal anti-inflammatory drugs show analgetic, anti-inflammatory and antipyretic activity; hence, they offer broad applications in the therapy of rheumatic diseases and other inflammatory disorders. Nonsteroidal anti-inflammatory drugs containing a carboxyl group irritate gastrointestinal tract; thus, their use is not recommended for patients with gastrointestinal tract diseases. Therefore, there is a significant demand for nonsteroidal anti-inflammatory drugs without the side effects mentioned above.

It is known that the oligomers of synthetic 3-hydroxybutyric acid are hydrolyzed by human enzymes and that their metabolites are non-toxic. Recent studies (*Nature* **400,** 173 -177 [1999]; *PNAS - Proc. Nat. Acad. Sci. USA* (Med. Sci) **97,** 5440-5444 [2000]) postulate that the therapy with 3-hydroxybutyric acid counteracts Alzheimer's disease. Esters of acetylsalicylic acid and poly-3-hydroxybutyric acid are also claimed to possess advantageous pharmacological effect (Polish patent application Jedlinski, Z., Juzwa, M., Kurek, A., Zawidlak, B. P-348 487 [2001] and European patent application EP 1404640 A1 (WO03004451 A1).

However, esters of oligo(3-hydroxybutyric acid) and nonsteroidal anti-inflammatory drugs (NSAIDs) listed above have not been known so far.

It has been found unexpectedly that it is possible to obtain novel esters of oligo(3-hydroxybutyric acid) with nonsteroidal anti-inflammatory drugs (NSAIDs) (including ibuprofen, ketoprofen, fenoprofen, naproxen, diclofenac, indomethacine, sulindac, tolmetin, fenoprofen, or salicylic acid) containing carboxyl groups without the use of toxic catalysts. Above mentioned nonsteroidal anti-inflammatory drugs prepared with the method according to the invention are linked with a non-toxic biocompatible polymer carrier by means of an ester bond, readily hydrolysable in biological conditions. The method of the invention enables the preparation of novel compounds that eliminate or reduce a wide range of side effects caused by mentioned above drugs and the preparation of drug delivery carrier systems with sustained activity.

Novel esters of nonsteroidal anti-inflammatory drugs with oligo(3-hydroxybutyric acid) obtained with the method of the invention possess the general formula **1,** where **R** is a radical of a nonsteroidal anti-inflammatory drug selected from the group consisting of ibuprofen, ketoprofen, naproxen, diclofenac, indomethacine, sulindac, tolmetin, fenoprofen or salicylic acid, n is a natural number from 2 to 35.

According to the invention method, a nonsteroidal anti-inflammatory drug selected from the group consisting of ibuprofen, diclofenac, ketoprofen, indomethacine, sulindac, naproxen, tolmetin, fenoprofen, or salicylic acid is converted, by means of a sodium or potassium hydride or hydroxide, into a sodium or potassium salt of the formula **3** in the presence of strongly polar solvents, preferably dimethyl sulfoxide (DMSO) or dimethyl formamide (DMF). Then these salts are reacted with racemic or optically active β-butyrolactone to produce an ester of oligo(3-hydroxybutyric acid).

The course of mentioned above syntheses is presented in the Scheme enclosed, where **X** and **R** have the above meaning, and **Y** is **H** or a hydroxyl group.

According to the inventory method, nonsteroidal anti-inflammatory drugs in the form of a sodium or potassium salt are subjected to the novel reaction with β-butyrolactone in the presence of strongly polar solvents, such as dimethyl sulfoxide (DMSO) or dimethyl formamide (DMF).

Novel esters of nonsteroid anti-inflammatory drugs with oligo(3-hydroxybutyric acid) possess the general formula **1,** where **R** is a radical of a nonsteroidal anti-inflammatory drug selected from the group consisting of ibuprofen of the formula **2a,** ketoprofen of the formula **2b,** naproxen of the formula **2c,** diclofenac of the formula **2d,** indomethacine of the formula **2e,** sulindac of the formula **2f,** tolmetin of the formula **2g,** fenoprofen of the formula **2h** or salicylic acid of the formula **2i.**

According to the invention, the method of obtaining novel nonsteroidal anti-inflammatory drugs of the formula **1,** where **n** and **R** have the above meaning, consists in converting a non-steroidal anti-inflammatory drug selected from the group containing ibuprofen, diclofenac, indomethacine, ketoprofen, naproxen, sulindac, tolmetin, fenoprofen or salicylic acid of the formula **2,** where **R** is a radical of a nonsteroidal anti-inflammatory drug, into a sodium or potassium salt of the formula **3,** where **R** has the above meaning, and **X** is a sodium or potassium atom, as well as in reacting the obtained sodium or potassium salt with racemic or optically active β-butyrolactone to produce appropriate esters of oligo(3-hydroxybutyric acid).

These salts are subjected next to the reaction with β-butyrolactone to open the β-butyrolactone ring in the position C-O to obtain an ester bond with the non-steroidal anti-inflammatory drug and to polymerize the lactone (see: Scheme). The compounds obtained are bonded by means of an ester bond with a biocompatible polymer carrier, consisting of oligomers of 3-hydroxybutyric acid containing 2-35 mers. The ester bond between the drug and the nanopolymer or oligomer of 3-hydroxybutyric acid is a bond hydrolysable in biological conditions.

Due to their lipophility and affinity to cell membranes, oligomers of 3-hydroxybutyric acid bonded with non-steroidal anti-inflammatory drugs by means of an ester bond give the novel pharmaceutical means specific features eliminating the irritation of the gastrointestinal tract mucosa. The polymer carrier, gradually hydrolysable in the gastrointestinal tract, facilitates the penetration of a drug through cell membranes.

The advantages of the novel esters of the invention and of the method to modify nonsteroidal anti-inflammatory drugs of the invention consist in the rapid drug transport through gastrointestinal tract as well as the reduction of drug toxicity. 3-hydroxybutyric acid and its oligomers released during the gradual hydrolysis are entirely nontoxic (Seebach, D., Brunner, A., Bachmann, B. M., Hoffmann, T., Kühnle, F. N. M., Lengweiler, U. D.: *"Biopolymers and -oligomers of (R)-3-Hydroxyalkanoic Acids - Contributions of Synthetic Organic Chemists"* Ernst Schering Research Foundation, Berlin,1995, **28).**

The compounds syntheses according to the above mentioned methods show non-toxicity what was examined in vitro and in vivo and can be used as anticancer and anti-inflammatory drugs showing better medical activity than the drugs not modified by nanopolymers of 3-hydroxybutyric acid.

The examples below illustrate the method of preparation of novel esters of nonsteroidal anti-inflammatory drugs with oligomers of 3-hydroxybutyric acid.

### Example 1

Into a dried reactor under argon atmosphere, 0.8640 g (0.036 mol) purified sodium hydride (NaH) and 8.2400 g (0.040 mol) ibuprofen was introduced and afterwards, 50 ml dried dimethyl sulfoxide (DMSO) was added. The reactor contents were stirred for 3 hours with a magnetic mixer at room temperature. Next into the reactor containing sodium salt of ibuprofen, β-butyrolactone in the amount of 9.288 g (0.108 mol) was introduced, and the reactor contents was stirred with a magnetic mixer. The conversion degree was analyzed on the basis of infrared (IR) spectroscopic analysis; band decay was noted for 1830 cm⁻¹ characteristic for the carbonyl carbon of the ester group in β-butyrolactone with another band appearing at 1735 cm⁻¹ characteristic for the carbonyl carbon of the ester groups in a polyester. When the conversion degree reached 100%, the solvent (DMSO) was evaporated under reduced pressure. Next, 40 ml acidified chloroform and 30 m! distilled water were introduced into the reaction mixture, and the product obtained was extracted with a chloroform-water mixture. After the two-phase system separation, the product of the organic (chloroform) phase was precipitated in hexane, and dried under reduced pressure to obtain a solid mass. 15.8784 g ester of ibuprofen and oligomer of 3-hydroxybutyric acid were obtained (yield: 95%). The molecular weight of the product obtained was determined by GPC method (based on calibration for narrow polystyrene standards); Mₙ = 450 [g/mol].

The analysis of end groups with ESI-MS mass spectrometry method (negative ions) revealed the presence of following molecular ions:
m/z = 377; [C₁₃H₁₇O₂(C₄H₆O₂)₂]⁻
m/z = 463; [C₁₃H₁₇O₂(C₄H₆O₂)₃]⁻
m/z = 544; [C₁₃H₁₇O₂(C₄H₆O₂)₄]⁻

### Example 2

The synthesis was performed using the same method as presented in Example 1. 3.3600 g (0.060 mol) potassium hydroxide (KOH), 13.6140 g (0.066 mol) ibuprofen, 80 ml DMSO, 30.9600 g (0.360 mol) β-butyrolactone were used. When the conversion degree reached 100%, the solvent was evaporated. Next 100 ml acidified chloroform and 50 ml distilled water were introduced into the reaction mixture to obtain 41.6046 g ester of ibuprofen and oligomer of 3-hydroxybutyric acid (yield: 96%). The molecular weight of the product obtained was determined by GPC method (based on calibration for narrow polystyrene standards); Mₙ = 740 [g/mol].

The analysis of end groups with ESI-MS mass spectrometry method (negative ions) revealed the presence of the following molecular ions:
m/z = 635; [C₁₃H₁₇O₂(C₄H₆O₂)₅]⁻
m/z = 721; [C₁₃H₁₇O₂(C₄H₆O₂)₆]⁻
m/z = 807; [C₁₃H₁₇O₂(C₄H₆O₂)₇]⁻

### Example 3

Into a dried reactor under argon atmosphere 11.4480 g (0.036 mol) sodium salt of diclofenac, 15.4800 g (0.180 mol) β-butyrolactone, and 85 mi dried dimethyl sulfoxide were introduced and afterwards, the reactor contents was stirred with a magnetic mixer at room temperature until the whole lactone was used to the reaction. The conversion degree was analyzed on the basis of infrared (IR) spectroscopic analysis; a band decay was noted for 1830 cm⁻¹ characteristic for the carbonyl carbon of the ester group in β-butyrolactone with another band appearing at 1735 cm⁻¹ characteristic for the carbonyl carbon of the ester groups in a polyester. When the conversion degree reached 100%, the solvent (DMSO) was evaporated under reduced pressure. Next, 75 ml acidified chloroform and 40 ml distilled water were introduced into the reaction mixture and product obtained was extracted with the chloroform-water mixture. After the two-phase system separation, the product of the organic (chloroform) phase was precipitated in hexane and dried under reduced pressure to obtain a solid mass. 27.3952 g ester of diclofenac and oligomer of 3-hydroxybutyric acid have been obtained (yield: 94%). The molecular weight of the product obtained was determined by GPC method (based on calibration for narrow polystyrene standards); Mₙ = 800 [g/mol].

The analysis of end groups with ESI-MS mass spectrometry method (negative ions) revealed the presence of the following molecular ions:
m/z = 748; [C₁₄H₁₀Cl₂NO₂(C₄H₆O₂)₅]⁻
m/z = 834; [C₁₄H₁₀Cl₂NO₂(C₄H₆O₂)₆]⁻
m/z = 920; [C₁₄H₁₀Cl₂NO₂(C₄H₆O₂)₄]⁻

### Example 4

The synthesis was performed in the manner presented in Example 3. There was used 6.3600 g (0.02 mol) sodium salt of diclofenac, 51.6000 g (0.60 mol) β-butyrolactone and 55 ml DMSO. When the conversion degree reached 100%, the solvent was evaporated. Next, into the reaction mixture there was introduced 120 ml acidified chloroform and 50 ml distilled water to obtain 56.3696 g ester of diclofenac and oligomer of 3-hydroxybutyric acid (yield: 98%). The molecular weight of the product obtained was determined with GPC method (on the basis of calibration for narrow polystyrene standards); Mₙ = 2950 [g/mol].

### Example 5

Into a dried reactor under argon atmosphere 3.3600 g (0.060 mol) potassium hydroxide (KOH), 16.7826 g (0.066 mol) ketoprofen were introduced and afterwards, 95 ml dried dimethyl sulfoxide (DMSO) was added. The reactor contents was stirred for 3 hours with a magnetic mixer at room temperature. Next, β-butyrolactone in the amount of 30.9600 g (0.360 mol) was introduced into the reactor containing potassium salt of ketoprofen, and the reactor contents was stirred with a magnetic mixer at room temperature until the whole lactone was subjected to the reaction. The conversion degree was analyzed on the basis of infrared (IR) spectroscopic analysis; a band decay was noted for 1830 cm⁻¹ characteristic for the carbonyl carbon of the ester group in β-butyrolactone with another band appearing at 1735 cm⁻¹ characteristic for the carbonyl carbon of the ester groups in a polyester. When the conversion degree reached 100%, the solvent (DMSO) was evaporated under reduced pressure. Next, 95 ml acidified chloroform and 50 ml distilled water were introduced into the reaction mixture, and the product obtained was extracted with a chloroform-water mixture. After the two-phase system separation, the product of the organic (chloroform) phase was precipitated in hexane and dried under reduced pressure to obtain a solid mass. 43.8900 g ester of ketoprofen and oligomer of 3-hydroxybutyric acid have been obtained (yield: 95%). The molecular weight of the product obtained was determined by GPC method (based on calibration for narrow polystyrene standards); Mₙ = 800 [g/mol].

The analysis of end groups with ESI-MS mass spectrometry method (negative ions) revealed the presence of the following molecular ions:
m/z = 683; [C₁₆H₁₃O₃(C₄H₆O₂)₅]⁻
m/z = 769; [C₁₆H₁₃O₃ (C₄H₆O₂)₆]⁻
m/z = 855; [C₁₆H₁₃O₃ (C₄H₆O₂)₇]⁻

### Example 6

Into a dried reactor under argon atmosphere, 2.5200 g (0.045 mol) potassium hydroxide (KOH), 10.9260 g (0.048 mol) naproxen were introduced and afterwards, 80 ml dried dimethyl formamide (DMF) was added. The reactor contents was stirred for 3 hours with a magnetic mixer at room temperature. Next, β-butyrolactone in the amount of 42.5700 g (0.495 mol) was introduced into the reactor containing potassium salt of naproxen and the reactor contents was stirred with a magnetic mixer until the whole lactone was subjected to the reaction. The conversion degree was analyzed on the basis of infrared (IR) spectroscopic analysis; a band decay was noted for 1830 cm⁻¹ characteristic for the carbonyl carbon of the ester group in β-butyrolactone with another band appearing at 1735 cm⁻¹ characteristic for the carbonyl carbon of the ester groups in a polyester. When the conversion degree reached 100%, the solvent (DMF) was evaporated under reduced pressure. Next, 100 ml acidified chloroform and 50 ml distilled water were introduced into the reaction mixture, and the product obtained in this way was extracted with a chloroform-water mixture. After the two-phase system separation, the product of the organic (chloroform) phase was precipitated in hexane and then dried under reduced pressure to obtain a solid mass. 51.3438 g ester of naproxen and oligomer of 3-hydroxybutyric acid were obtained (yield: 97%). The molecular weight of the product obtained was determined by GPC method (based of calibration for narrow polystyrene standards); Mₙ = 1200 [g/mol].

The analysis of end groups with ESI-MS mass spectrometry method (negative ions) revealed the presence of the following molecular ions:
m/z = 1089; [C₁₄H₁₃O₃(C₄H₆O₂)₁₀]⁻
m/z = 1175; [C₁₄H₁₃O₃ (C₄H₆O₂)₁₁]⁻
m/z = 1261; [C₁₄H₁₃O₃ (C₄H₆O₂)₁₂]⁻

### Example 7

The synthesis was performed in the manner presented in Example 6. There was used 3.200 g (0.080 mol) sodium hydroxide (NaOH), 20.2400 g (0.088 mol) naproxen, 100 ml DMSO, 20.64 g (0.240 mol) β-butyrolactone. When the conversion degree reached 100%, the solvent was evaporated. Next, into the reactor there was introduced 110 ml acidified chloroform and 50 ml distilled water to obtain 36.6976 g ester of naproxen and oligomer of 3-hydroxybutyric acid (yield: 94%). The molecular weight of the product obtained was determined with GPC method (on the basis of calibration for narrow polystyrene standards); Mₙ = 500 [g/mol].

The analysis of end groups with ESI-MS mass spectrometry method (negative ions) revealed the presence of molecular ions:
m/z = 401; [C₁₄H₁₃O₃(C₄H₆O₂)₂]⁻
m/z = 487; [C₁₄H₁₃O₃ (C₄H₆O₂)₃]⁻
m/z = 573; [C₁₄H₁₃O₃ (C₄H₆O₂)₄]⁻

### Example 8

Into a dried reactor under argon atmosphere, 1.8000 g (0.045 mol) sodium hydroxide (NaOH) and 17.1078 g (0.048 mol) sulindac were introduced and afterwards, 80 ml dried dimethyl sulfoxide (DMSO) was added. The reactor contents was stirred for 3 hours with a magnetic mixer at room temperature. Next, β-butyrolactone in the amount of 11.6100 g (0.135 mol) was introduced into the reactor containing sodium salt of sulindac and the reactor contents was stirred with a magnetic mixer until the whole lactone was subjected to the reaction. The conversion degree was analyzed on the basis of infrared (IR) spectroscopic analysis; a band decay was noted for 1830 cm⁻¹ characteristic for the carbonyl carbon of the ester group in β-butyrolactone with another band appearing at 1735 cm⁻¹ characteristic for the carbonyl carbon of the ester groups in a polyester. When the conversion degree reached 100%, the solvent (DMSO) was evaporated under reduced pressure. Next, 70 ml acidified chloroform and 40 ml distilled water were introduced into the reaction mixture, and the product obtained was extracted with a chloroform-water mixture. After the two-phase system separation, the product of the organic (chloroform) phase was precipitated in hexane, and then dried under reduced pressure to obtain a solid mass. 25.7130 g ester of sulindac and oligomer of 3-hydroxybutyric acid were obtained (yield: 93%). The molecular weight of the product obtained was determined by GPC method (based onf calibration for narrow polystyrene standards); Mₙ = 600 [g/mol].

The analysis of end groups with ESI-MS mass spectrometry method (negative ions) revealed the presence of the following molecular ions:
m/z = 527; [C₂₀H₁₆FO₃S(C₄H₆O₂)₂]⁻
m/z = 613; [C₂₀H₁₆FO₃S(C₄H₆O₂)₃]⁻
m/z = 699; [C₂₀H₁₆FO₃S(C₄H₆O₂)₄]⁻

### Example 9

The synthesis was performed in the manner presented in Example 8. There was used 2.24000 g (0.040 mol) potassium hydroxide (KOH), 15.6820 g (0.044 mol) sulindac, 150 ml DMF, 44.7200 g (0.52 mol) β-butyrolactone. When the conversion degree reached 100%, the solvent was evaporated. Next, into the reactor there was introduced 120 ml acidified chloroform and 70 ml distilled water to obtain 59.2128 g ester of sulindac and oligomer of 3-hydroxybutyric acid (yield: 96%). The molecular weight of the product obtained was determined with GPC method (on the basis of calibration for narrow polystyrene standards); Mₙ = 1500 [g/mol].

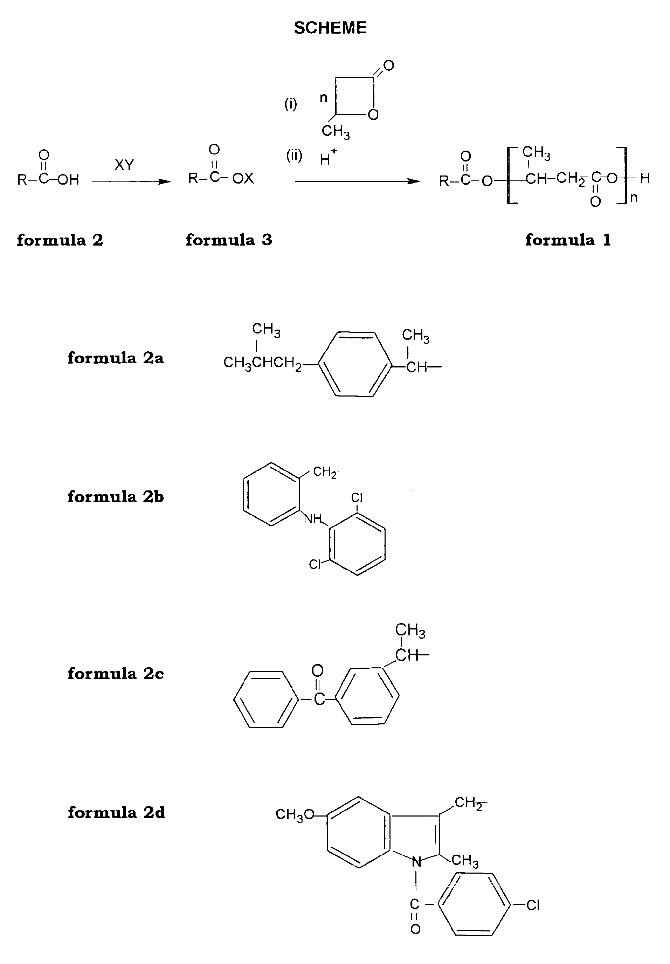

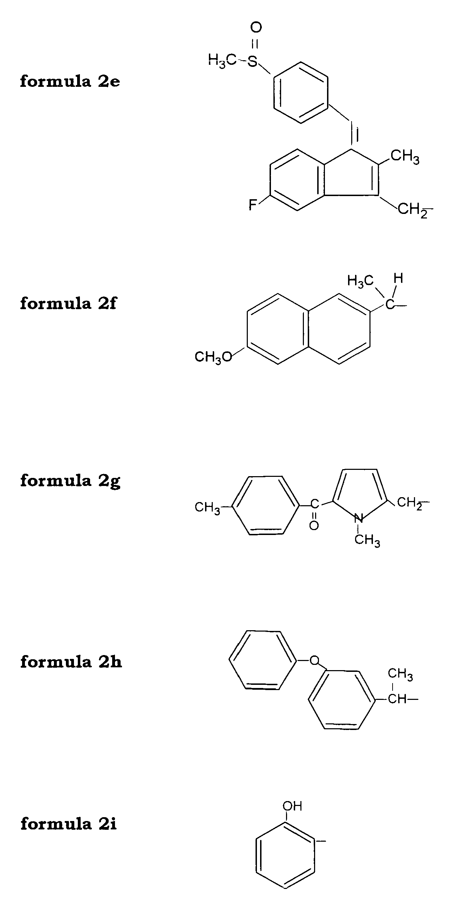

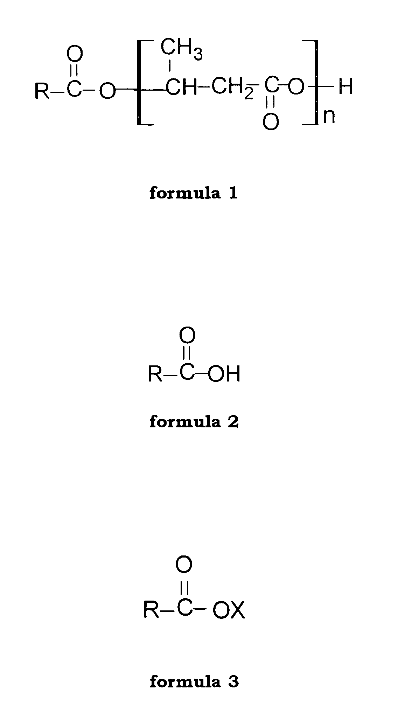

## Claims

1. According to the invention, novel esters of nonsteroidal anti-inflammatory drugs and oligo(3-hydroxybutyric acid) possessing the general formula 1, where R is a radical of a nonsteroidal anti-inflammatory drug selected from the group consisting of ibuprofen, ketoprofen, naproxen, diclofenac, indomethacine, sulindac, tolmetin, fenoprofen or salicylic acid, where n from 2 to 35.

2. The method for preparation of esters of nonsteroidal anti-inflammatory drugs, **characterized in that** converting the compound of the formula 2, where R is a radical of a nonsteroidal anti-inflammatory drug selected from the group consisting of ibuprofen, diclofenac, indomethacine, ketoprofen, naproxen, sulindac, tolmetin, fenoprofen or salicylic acid, into a sodium or potassium salt of the formula 3, where R has the above meaning, and X is a sodium or potassium atom as well as in reacting the sodium or potassium salt obtained with racemic or optically active β-butyrolactone to produce an ester of oligo(3-hydroxybutyric acid).

3. The method as mentioned in point 1, 2 the above mentioned compounds of the formula 2 **characterized in that** converting the into sodium or potassium salt of the formula 3 by means of a sodium or potassium hydride or hydroxide in the presence of a strongly polar non-toxic solvent, preferably dimethyl sulfoxide (DMSO) or dimethylformamide (DMF) and in subjecting the obtained salts to the reaction with β-butyrolactone to open the lactone ring and to polymerize the lactone.
